# EUROPEAN PATENT APPLICATION

(11) **EP 4 467 159 A1**
(43) Date of publication of application: **27.11.2024**
(21) Application number: 23175612.3
(22) Date of filing: 26.05.2023
(51) Int. Cl.: A61K 47/10, A61K 9/06, A61K 9/08

(54) **(4R)-3-(4-FLUORO-2-HYDROXYPHENYL)-4-METHYL-4,5-DIHYDRO-1HPYRAZOLE- 1-CARBOXIMIDAMIDE HYDROCHLORIDE AND DERMAL FORMULATIONS THEREOF**

(71) Applicant: AnaMar AB, 111 36 Stockholm (SE)
(72) Inventor: PETTERSSON, Lars, 224 80 LUND (SE)
(74) Representative: EIP

(57) **Abstract**

The present invention relates to the compound (4R)-3-(4-fluoro-2-hydroxyphenyl)-4-methyl-4,5-dihydro-1H-pyrazole-1-carboximidamide hydrochloride, to a dermal formulation comprising the compound and one or more pharmaceutically acceptable excipients or carriers, and to the use of the compound as a medicament, particularly for use in the treatment of skin fibrosis.

## Description

### Field of the invention

The present invention relates to (4R)-3-(4-fluoro-2-hydroxyphenyl)-4-methyl-4,5-dihydro-1H-pyrazole-1-carboximidamide hydrochloride and aqueous dermal formulations thereof for topical treatment of skin fibrosis.

### Background of the invention

Preparation of 5-HT_{2B} receptor antagonists as anti-fibrotic agents was described in WO2016/207231A1. Potent skin and lung anti-fibrotic effects were demonstrated in mice after oral administration, and compounds were selected for pharmaceutical development. One such selected compound was 3-(3-fluoro-2-hydroxyphenyl)-4-methyl-4,5-dihydro-1H-pyrazole-1-carboximidamide, exemplified by its hydrobromide salt (Example 50 in WO2016/207231A1).

Skin fibrosis, like other fibrotic conditions, is initiated by tissue injury and/or inflammation and is a natural response of wound healing. Three phases characterize wound healing, namely inflammation, proliferation, and the remodeling phase. TGF-β is a key regulator in the remodeling phase enabling the differentiation of fibroblasts to myofibroblasts, producing extracellular matrix (ECM) proteins, e.g. collagen and fibronectin, to assist with wound coverage. Prolonged inflammation, as in burn injuries, mechanical tension, and other perturbing factors, result in excessive production of ECM proteins and abnormal scarring of the skin, the keloid scars and hypertrophic scars.

Excessive scar formation may lead to impaired aesthetics, impaired physical function, and psychological disorders. Currently there are no standard treatments of keloids and no satisfactory treatments of hypertrophic scars (Hofmann, E. et al. Biomedicines 2023, 11, 1056). There is a very high unmet medical need for new treatment options of fibrotic disorders of the skin.

Dermal delivery of pharmaceutical compounds for the topical treatment of dermal conditions may provide certain advantages, such as high local concentration of the active compound in the skin, while systemic exposure, possibly leading to adverse effects, can be minimized. Skin penetration is, however, highly dependent on physical-chemical properties of the active compound; the main hurdle being the penetration of the *stratum corneum,* the keratinized superficial skin layer. Especially, polar compounds are not able to adequately penetrate skin by passive formulation methods (Nikoli'c, I. et al. Pharmaceutics 2022, 14, 1144) but good aqueous solubility may help increasing the compound exposure once penetrated the hydrophilic epidermis and dermis. Ideally, for topical treatment, the undesired transdermal permeation with possible systemic exposure should be minimized. The penetration of a compound through *stratum corneum* into epidermis and dermis, and permeation through the skin, can be measured *in vitro* using porcine or human skin, since results from these membranes are known to correlate with human *in vivo* data (Iliopoulos F. et al. Front. Drug. Deliv. (2022) 2:1049848).

### Summary of the invention

There are currently no truly antifibrotic medical treatment options for excessive scar formation of the skin. Topical treatment with anti-fibrotic agents can meet this huge medical need.

An object of the present application is to find suitable anti-fibrotic compounds for the preparation of aqueous dermal formulations providing adequate skin penetration with little permeability through the skin. A further objective is to provide such aqueous dermal formulations for the treatment of skin fibrotic conditions.

The above objects are achieved by the subject matter described herein.

In the present disclosure, the compound (R)-3-(4-fluoro-2-hydroxyphenyl)-4-methyl-4,5-dihydro-1H-pyrazole-1-carboximidamide hydrochloride was identified as a stable compound with good solubility in water and other excipient solvents. The compound, formulated as an aqueous formulation and applied on skin, displayed good skin penetration properties with very little transdermal permeability.

Various aspects of the invention are set out in the attached independent claims.

Further advantageous embodiments of the invention are set out in the attached dependent claims.

Applications and advantages of aspects and embodiments of the invention will be apparent from the following detailed description.

### Detailed description of the invention

The anti-fibrotic compounds described in WO2016/207231A1 considered for development, e.g. Example 50 in WO2016/207231A1 (LogD -0.7), are highly polar compounds. Consequently, for dermal applications the base form (Compound 1), with expected reduced polarity, was selected for dermal formulation studies. After comparing solubilities in excipient solvents of Compound 1 with the corresponding enantiomeric base (Compound 2), isolated by chiral supercritical fluid chromatography (SFC), it was surprisingly found that Compound 2 was considerably more soluble in water, by a factor of 10-20, and consequently more suitable for aqueous formulations.

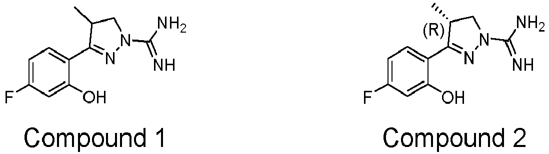

The 3D-structures and absolute stereochemistry of the two new enantiomeric compounds, (4R)- and (4S)-3-(4-fluoro-2-hydroxyphenyl)-4-methyl-4,5-dihydro-1H-pyrazole-1-carboximidamide were determined by X-ray crystallography (XRD) of the corresponding HCl salts, respectively.

Initial formulation and skin *in vitro* permeation tests (IVPT) using pig ear skin were performed with Compound 2 (3%, w/w) in a vehicle solvent mixture, based on solubility data, consisting of water (50%), PEG-400 (25%) and propylene glycol (25%). Compound 2 solubilities in the separate solvents were found to be 10 mg/mL in water, 63 mg/mL in PEG-400, and 35 mg/mL in propylene glycol. The solubility of Compound 2 in the vehicle solvent mixture was found to be 7.5% (w/w).

The results showed surprisingly good penetration into epidermis and dermis with only small transdermal delivery to the receiver fluid. Unfortunately, stability studies of the separate solvent solutions of Compound 2, and of the formulation, detected a slow formation of impurities.

Despite its enhanced polar property, the available HCl salt of Compound 2, (4R)-3-(4-fluoro-2-hydroxyphenyl)-4-methyl-4,5-dihydro-1H-pyrazole-1-carboximidamide hydrochloride (Compound 3), was investigated to possibly overcome the stability issue.

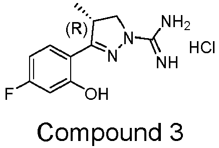

The same test procedures, and the same solvent formulation, as performed for Compound 2 were used to evaluate Compound 3. Good solubilities were demonstrated (34 mg/mL in water, 16 mg/mL in PEG-400, and 51 mg/mL in propylene glycol, and 7.1% (w/w) in the vehicle solvent mixture). The IVPT results clearly showed, like for Compound 2, surprisingly good penetration into epidermis (7.7 mg/cm²) and dermis (4.0 mg/cm²) with only small transdermal delivery to the receiver fluid (0.38 mg/cm²). In addition, Compound 3 was found stable in separate solvents during the stability studies and in the mixed solvent formulation. The Compound 3 formulation also showed a slightly acidic pH (pH 5.5), favorable for dermal formulations, while the Compound 2 formulation was found basic (pH 10.1).

According to a first aspect of the invention, the above mentioned and other objects are achieved by the compound (4R)-3-(4-fluoro-2-hydroxyphenyl)-4-methyl-4,5-dihydro-1H-pyrazole-1-carboximidamide hydrochloride (the compound of Formula 3).

The inventive compound is preferably provided in an enantiomerically pure or enantiomerically enriched form. In some embodiments, the compound has an enantiomeric purity of at least 80%, preferably at least 90%, and more preferably at least 95%.

As used herein, the term "enantiomeric purity" refers to the prevalence of one enantiomer of a compound over the opposite enantiomer of the compound. A typical enantiomerically pure compound comprises greater than about 80% by weight of one enantiomer of the compound and less than about 20% by weight of the opposite enantiomer of the compound, more preferably greater than about 90% by weight of one enantiomer of the compound and less than about 10% by weight of the opposite enantiomer of the compound, even more preferably greater than about 95% by weight of one enantiomer of the compound and less than about 5% by weight of the opposite enantiomer of the compound, and most preferably greater than about 97% by weight of one enantiomer of the compound and less than about 3% by weight of the opposite enantiomer of the compound.

The inventive compound has been found to be useful in dermal formulations and particularly in aqueous dermal formulations.

According to a second aspect of the invention, there is provided a dermal formulation comprising the compound (4R)-3-(4-fluoro-2-hydroxyphenyl)-4-methyl-4,5-dihydro-1H-pyrazole-1-carboximidamide hydrochloride (the compound of Formula 3) and one or more pharmaceutically acceptable excipients or carriers.

The inventive dermal formulation preferably comprises (4R)-3-(4-fluoro-2-hydroxyphenyl)-4-methyl-4,5-dihydro-1H-pyrazole-1-carboximidamide hydrochloride in an enantiomerically pure or enantiomerically enriched form.

In some embodiments of the second aspect at least 80%, preferably at least 90%, and more preferably at least 95%, of the total content of 3-(4-fluoro-2-hydroxyphenyl)-4-methyl-4,5-dihydro-1H-pyrazole-1-carboximidamide hydrochloride in the formulation consists of (4R)-3-(4-fluoro-2-hydroxyphenyl)-4-methyl-4,5-dihydro-1H-pyrazole-1-carboximidamide hydrochloride.

As used herein, the term "dermal formulation" refers to a composition or mixture specifically designed and formulated for topical application on the skin. It is intended to deliver active ingredients, provide therapeutic effects, enhance skin health, or improve cosmetic appearance through direct contact with the skin.

The term "dermal formulation" encompasses various types of formulations, including creams, lotions, gels, ointments, sprays, foams, patches, or any other form suitable for application to the skin. These formulations typically consist of a combination of active ingredients, excipients, vehicles, penetration enhancers, emulsifiers, stabilizers, and other components that contribute to the desired properties, stability, and efficacy when applied topically.

In some embodiments, the dermal formulation is an aqueous formulation. As used herein, the term "aqueous formulation" refers to a formulation, composition or mixture that contains water as the primary solvent or dispersion medium.

In some embodiments, the dermal formulation comprises ≥ 20 % (w/w) of water, preferably ≥ 30 % (w/w) of water, and more preferably ≥ 40 % (w/w) of water.

In some embodiments, the dermal formulation comprises 50-98 % (w/w) of water, preferably 50-95 % (w/w) of water, and more preferably 50-90 % (w/w) of water.

In some embodiments, the dermal formulation further comprises 1-30 % (w/w) propylene glycol, preferably 5-25% (w/w) propylene glycol.

In some embodiments, the dermal formulation comprises or consists of a solution or suspension of the inventive compound in a liquid, preferably aqueous, carrier. The solution or suspension may be designed for topical application on the skin or mucous membranes. The solution or suspension may be specifically formulated to provide therapeutic effects when applied to the affected area.

In some embodiments, the dermal formulation is a cream. As used herein, the term "cream" refers to a semisolid formulation designed for topical application on the skin or mucous membranes, containing the inventive compound suspended or dissolved in a suitable base or vehicle. The cream is specifically formulated to provide therapeutic effects when applied to the affected area. Methods and components for formulating dermal cream formulations are known and readily available to the skilled person.

In some embodiments, the dermal formulation is a gel. As used herein, the term "gel", as described in patent language, refers to a semisolid formulation designed for topical or mucosal application, characterized by its gel-like consistency and ability to retain shape under minimal shear stress. The gel composition comprises the inventive compound and a gelling agent or matrix, which imparts the desired gel-like properties to the formulation. Methods and components for formulating dermal gel formulations are known and readily available to the skilled person.

According to a third aspect of the invention, there is provided the compound (4R)-3-(4-fluoro-2-hydroxyphenyl)-4-methyl-4,5-dihydro-1H-pyrazole-1-carboximidamide hydrochloride (the compound of Formula 3) for use as a medicament.

According to a fourth aspect of the invention, there is provided the compound (4R)-3-(4-fluoro-2-hydroxyphenyl)-4-methyl-4,5-dihydro-1H-pyrazole-1-carboximidamide hydrochloride (the compound of Formula 3) for use in the treatment of skin fibrosis.

In some embodiments, the skin fibrosis is linked to or caused by a disease or skin inflammation, a natural ageing process, scarring caused by burn injuries, by surgery, by plastic surgery, or by other skin injuries, acne, or keloids.

In some embodiments, the skin fibrosis is linked to or caused by the disease systemic sclerosis.

In some embodiments, the skin fibrosis is linked to or caused by burn injuries.

In some embodiments, the skin fibrosis is linked to or caused by surgery or plastic surgery.

In some embodiments, the skin fibrosis is linked to or caused by acne.

In some embodiments, the skin fibrosis is linked to or caused by keloids.

According to another aspect of the invention, there is provided the use of the compound (4R)-3-(4-fluoro-2-hydroxyphenyl)-4-methyl-4,5-dihydro-1H-pyrazole-1-carboximidamide hydrochloride (the compound of Formula 3) in the manufacture of a medicament useful in treatment of skin fibrosis.

According to another aspect of the invention, there is provided a method of treating skin fibrosis comprising administering a therapeutically effective amount of the compound (4R)-3-(4-fluoro-2-hydroxyphenyl)-4-methyl-4,5-dihydro-1H-pyrazole-1-carboximidamide hydrochloride (the compound of Formula 3) to a patient in need thereof.

### Examples

### Example 1

### 3-(4-fluoro-2-hydroxyphenyl)-4-methyl-4,5-dihydro-1H-pyrazole-1-carboximidamide hydrochloride

The racemic HCl salt was prepared by methods analogous to methods described in WO2016/207231A1.

¹H-NMR ((CD₃)₂SO) d 1.16 (d, 3H), 3.69 (dd, 1H), 4.04-4.17 (m, 2H), 6.77 (dt, 1H), 6.88 (dd, 1H), 7.76 (dd, 1H), 7.89 (broad s, 4H), 10.61 (broad s, 1H).

### Example 2

### (4R)-3-(4-fluoro-2-hydroxyphenyl)-4-methyl-4,5-dihydro-1H-pyrazole-1-carboximidamide (Compound 2)

The enantiomers of the racemic 3-(4-fluoro-2-hydroxyphenyl)-4-methyl-4,5-dihydro-1H-pyrazole-1-carboximidamide were separated by chiral supercritical fluid chromatography (SFC).

Conditions: Lux A1 (21.2 mm x 250 mm, 5 µm, column Temperature 40 °C, flow Rate 50 mL/min, 100 BarG, isocratic conditions 30:70 MeOH:CO₂ (0.2% v/v NH₃). 3-(4-Fluoro-2-hydroxyphenyl)-4-methyl-4,5-dihydro-1H-pyrazole-1-carboximidamide hydrochlorid (5.94 g) was dissolved to 40 mg/mL in MeOH:CH₂Cl₂ (1:1) and purified by SFC. The eluates were evaporated, providing the R- and S-enantiomers, respectively, in base forms, both with ee (enantiomeric excess) 100.

¹H-NMR (CDsOD) d 1.24 (d, 3H), 3.58 (dd, 1H), 3.97 (t, 1H), 4.19 (m, 1H), 6.19 (dt, 1H), 6.38 (dd, 1H), 7.33 (t, 1H).

### Example 3

### (4R)-3-(4-fluoro-2-hydroxyphenyl)-4-methyl-4,5-dihydro-1H-pyrazole-1-carboximidamide hydrochloride (Compound 3)

A solution of HCl in MeOH (sat.) was added to a solution of (4R)-3-(4-fluoro-2-hydroxyphenyl)-4-methyl-4,5-dihydro-1H-pyrazole-1-carboximidamide in MeOH. The solution was then concentrated at reduced pressure and the residue was dried in vacuum at 40 °C to give the title compound (40% from racemic HCl salt, ee 100).

NMR as for racemate.

X-ray diffraction analysis (XRD) was used to determine the absolute configuration (4R). Data were collected at 100 K at Diamond Light Source, Didcot, England (λ = 0.7000 Å), equipped with a Pilatus 6M-F detector. The structure (at 0.75 Å resolution) was solved using SHELXS2 and refined using SHELXL2 in combination with the graphical user interface SHELXLE3.

### Example 4

### Solubility of Compound 1 and Compound 2 in water

The aq. solubility was determined by the addition of an excess of solid compound to MQ-water. The solution and solid were mixed for 24 h at room temperature to ensure equilibrium. The excess solid compound was removed by filtration. The filtrate was then diluted and quantified with LC-UV against a 3-point calibration curve of the actual compound.

Compound 1 aq. solubility: 0.9 mg/mL

Compound 2 aq. solubility: 20.1 mg/mL

The results show a much better solubility (>20x) of the enantiomeric Compound 2, compared with the racemic Compound 1 in MQ-water after 24 h of equilibration.

### Example 5

### Solubility and stability of Compound 2 and Compound 3 in excipient solvents, including water

Excess of each substance were added to the solvents. The samples were then stirred for 1 hour and left at room temperature overnight in the dark. The samples were centrifuged, and supernatants were transferred to vials and the compound concentrations were analysed by HPLC.

The samples were then stored in the dark at ambient temperature and analysed after one day, and after two and four weeks of storage to assess the stability of the compound solutions, i.e. detection of related impurities.

The solubilities (mg/mL) of Compound 2 in the following solvents were:
10.5 in HPLC water; 4.21 in EtOH; 36.5 in propylene glycol ; >63* in PEG-400;
0.21 in caprylic/capric triglyceride; >15* in glycerol; 1.9 in DMI; >51* in transcutol

The solubilities (mg/mL) of Compound 3 in the following solvents were:
33.8 in HPLC water; 21.5 in EtOH; >51* in propylene glycol; 16.5 in PEG-400; 0.01 in caprylic/capric triglyceride; >34* in glycerol; 0.05 in DMI; 10.3 in transcutol. (* all material dissolved)

The stability was not investigated in caprylic/capric triglycerides and in dimethyl isosorbide (DMI) as the solubilities were low, and not in transcutol since degradation was seen already at the initial analysis at 24 h for both compounds (2.91% for Compound 2 and 0.27% for Compound 3).

The detected related impurities (%) in Compound 2 solutions were at 24 h / 2 weeks / 4 weeks in
HPLC water: 0.13/0.54/0.84
EtOH: 0.24 / 0.54 / 0.69
Propylene glycol: 0.16 / 0.23 / 0.30
PEG-400: 0.18 / 0.39 / 0.50
Glycerol: 0.30 / 0.57 / 0.75

The detected related impurities (%) in Compound 3 solutions were at 24 h / 2 weeks / 4 weeks in
HPLC water: 0.10/0.08/0.08
EtOH: 0.10/0.07 / 0.08
Propylene glycol: 0.10 / 0.08 / 0.09
PEG-400: 0.10 / 0.08 / 0.09
Glycerol: 0.13 / 0.12 / 0.13

The results show that a slow but steady degradation is seen in all Compound 2 solutions, while all the Compound 3 solutions are stable during four weeks of storage.

### Example 6

### In vitro permeation tests (IVPT) of Compound 2 and Compound 3 in solvent formulations (donor vehicle).

Dermatomed skin membranes from pig inner ears were used as membranes in the Bronaugh diffusion cell equipment. Compounds were dissolved (3% w/w) in the donor vehicle consisting of 50% water, 25% PEG-400 and 25% propylene glycol. Prior to the experiment the solubilities of Compound 2 (7.5% w/w) and Compound 3 (7.1 % w/w) were determined by HPLC. Also, the pH of the formulations (pH 10.1 for Compound 2 formulation and pH 5.5 for Compound 3 formulation) was measured prior to application. The receptor solution consisted of a PBS buffer solution (pH 7.4). The donor solution was applied in an infinite dose (150 mg) with occlusion and sampling timepoints, to measure cumulative permeation, were 6, 12, 18, and 24 h.

At 24 h the membranes were cleaned, and the epidermis and dermis separated. The compounds were extracted with a water/acetonitrile mixture (50:50) and analyzed by HPLC.

### Results

The cumulative permeation (µg/cm², compound concentration in receptor solution measured by HPLC) were:
Compound 2 - 0.00 (6 h), 0.08 (12 h), 0.22 (18 h), 0.53 (24 h)
Compound 3 - 0.02 (6 h), 0.05 (12 h), 0.17 (18 h), 0.38 (24 h)

### Amount of compounds in the skin membranes

Compound 2 in epidermis: 991 µg/g, 7.9 µg/cm²
Compound 2 in dermis: 82.5 µg/g, 4.7 µg/cm²
Compound 3 in epidermis: 961 µg/g, 7.7 µg/cm²
Compound 3 in dermis: 64.7 µg/g, 4.0 µg/cm²

The results show that the permeation and accumulation in the tissue were similar for both compounds and no significant difference could be observed. After 24 h, >95% of the compound amounts remained in the skin tissue, while <5% permeated to the receptor solution.

The lower pH of the formulation with Compound 3 (pH 5.5) is better for skin applications. In addition, while the formulation of Compound 3 was stable, a relatively large degradation peak was found in the formulation of Compound 2, also previously detected in aqueous solutions in the stability study.

### Example 7

### Compound 3 (2% w/w) aqueous cream formulation. Batch size 100 q.

Aqueous phase: Polysorbate 60 (0.97 g) was heated to 50 °C and mixed with propylene glycol (15.00 g), glycerol (10.00 g), water (53.65 g) and Compound 3 (2.00 g) and stirred at 70 °C to a homogenous solution.

Lipid phase: A mixture of sorbitane monostearate (1.29 g), cetyl palmitate (1.93 g), cetostearyl alcohol (6.45 g) and 2-octyl-1 dodecanol (Kollicream OD) (8.71 g) was stirred at 70 °C to a homogenous solution.

The lipid phase was slowly added to the polar phase at 70 °C while stirring intensively. The mixture was then homogenised at 70 °C to give a homogenous emulsion, which was cooled to room temperature with moderate stirring.

### Example 8

### Compound 3 (2% w/w) aqueous gel formulations. Batch size 100 g.

A mixture of EDTA (0.05 g), benzalkonium chloride (0.005 g) and water was stirred at 60°C, then Carbopol 974 P (0.90 g) was added during vigorous stirring at 60°C. The mixture was cooled and stirred at room temperature for 45 minutes. Sodium hydroxide (0.30 g) was added, and the mixture was stirred for 30 minutes. Compound 3 (2.00 g) was mixed with propylene glycol (15.00 g) and added to the gel mixture, which was stirred and mixed to a homogenous formulation.

## Claims

1. The compound (4R)-3-(4-fluoro-2-hydroxyphenyl)-4-methyl-4,5-dihydro-1H-pyrazole-1-carboximidamide hydrochloride.

2. A compound according to claim 1, wherein the compound has an enantiomeric purity of at least 80%, preferably at least 90%, and more preferably at least 95%.

3. A dermal formulation comprising the compound according to any one of claims 1-2 and one or more pharmaceutically acceptable excipients or carriers.

4. The dermal formulation according to claim 3, wherein the dermal formulation is an aqueous formulation.

5. The dermal formulation according to any one of claims 3-4, wherein the dermal formulation comprises ≥ 20 % (w/w) of water, preferably ≥ 30 % (w/w) of water, and more preferably ≥ 40 % (w/w) of water.

6. The dermal formulation according to any one of claims 3-5, wherein the dermal formulation comprises 50-98 % (w/w) of water, preferably 50-95 % (w/w) of water, and more preferably 50-90 % (w/w) of water.

7. The dermal formulation according to any one of claims 3-6, wherein the dermal formulation further comprises 1-30 % (w/w) propylene glycol, preferably 5-25% (w/w) propylene glycol.

8. The dermal formulation according to any one of claims 3-7, wherein the dermal formulation is a cream.

9. The dermal formulation according to any one of claims 3-7, wherein the dermal formulation is a gel.

10. The compound according to any one of claims 1-2 for use as a medicament.

11. The compound according to any one of claims 1-2 for use in the treatment of skin fibrosis.

12. The compound for use according to claim 11, wherein the skin fibrosis is linked to or caused by a disease or skin inflammation, a natural ageing process, scarring caused by burn injuries, by surgery, by plastic surgery, or by other skin injuries, acne, or keloids.

13. The compound for use according to claim 11, wherein the skin fibrosis is linked to or caused by the disease systemic sclerosis.

14. The compound for use according to claim 11, wherein the skin fibrosis is linked to or caused by burn injuries.

15. The compound for use according to claim 11, wherein the skin fibrosis is linked to or caused by surgery or plastic surgery.

16. The compound for use according to claim 11, wherein the skin fibrosis is linked to or caused by acne.

17. The compound for use according to claim 11, wherein the skin fibrosis is linked to or caused by keloids.

18. Use of a compound according to any one of claims 1-2 in the manufacture of a medicament useful in treatment of skin fibrosis.

19. A method of treating skin fibrosis comprising administering a therapeutically effective amount of a compound according to any one of claims 1-2 to a patient in need thereof.
